# EUROPÄISCHE PATENTSCHRIFT

(11) **EP 2 578 187 B1**
(45) Veröffentlichungstag und Bekanntmachung des Hinweises auf die Patenterteilung: **20.07.2016**
(21) Anmeldenummer: 12006901.8
(22) Anmeldetag: 04.10.2012
(51) Int. Cl.: A61F 5/56, A61C 7/36

(54) **Langzeitretentionsvorrichtung**
Long term retention device
Dispositif de rétention à long terme

(30) Priorität: 04.10.2011 DE 102011114637
(43) Veröffentlichungstag der Anmeldung: 10.04.2013
(73) Patentinhaber: Weber, Joachim, 67071 Ludwigshafen (DE)
(72) Erfinder: Weber, Joachim, 67071 Ludwigshafen (DE)
(74) Vertreter: Otten, Roth, Dobler & Partner mbB Patentanwälte

(56) Entgegenhaltungen:
- DE-A1- 10 042 049
- US-A- 5 848 891
- US-A1- 2005 028 826

## Beschreibung

Die Erfindung betrifft eine kieferorthopädische Behandlungseinheit, insbesondere eine Langzeitretentionsvorrichtung zum Einstellen und Erhalt einer korrekten dentalen Bisslage von Oberkiefer und Unterkiefer bei schwierig oder gar nicht korrekt einzustellender condylärer Zentrik.

### Stand der Technik

Es sind mehrere derartige kieferorthopädische Geräte zur aktiven Behandlung bekannt, welche zur temporären Befestigung an Oberkieferzähnen eine Widerlagerkonstruktion und zur temporären Befestigung an Unterkieferzähnen eine Korrekturkonstruktion aufweisen. Alternativ werden auch adaptierbare Rampen angebracht, die dann nur in einem Kiefer nötig sind. In noch weiterem Umfang dienen kieferorthopädische Hilfsteile, die an festsitzenden Apparaturen angebracht werden (sog. Intermaxilläre Gummizüge, Derivate der Herbstapparatur usw.) und herausnehmbare funktionskieferorthopädische Geräte denselben Therapiezweck.

Die Lagerkonstruktionen zeichnen sich dabei vor allem durch Adaptationsmöglichkeiten während der Behandlung aus. Zudem sind sie für den temporären Einsatz bestimmt. Den Apparaturen ist gemein, dass sie während des Mundschlusses die Zahnreihen in eine korrekte Bisslageposition führen. Ist dies durch muskuläre Verspannung oder durch Limitation nicht möglich, so muss gegebenenfalls schrittweise die richtige Position durch Veränderung der Apparatur herbeigeführt werden. Durch den Einsatz von konfektionierten Fertigungsteilen entstehen dabei Ecken, Kanten und Nischen. Diese sind erschwert zu reinigen und führen häufig zu Scheuer- oder Druckstellen am anliegenden Weichgewebe.

Die Geräte führen in einem ersten Schritt vor allem zu einer muskulären Veränderung, wobei der Unterkiefer ausgehend von einer ungewünschten diagnostischen Lage in eine gewünschte therapeutische Lage gehalten wird. Bei den bisher neuesten bekannten aktiven Behandlungseinheiten lässt sich eine festsitzende bimaxilläre, paarig anzubringende kieferorthopädischorthodontische Apparatur zur orthopädischen Ventralverlagerung des Unterkiefers sowie Wachstumshemmung des Oberkiefers und/oder orthodontischen Bewegung der Molaren bzw. Seitenzähne im Ober- und Unterkiefer verwirklichen, wobei der justierbare Führungsbügel zusammen mit dem unterkieferseitigen Korrekturglied im Rahmen des Kieferschlusses als krafterzeugende Elemente wirken und eine effektive Ventralentwicklung des Unterkiefers durch eine aktive Führung in die therapeutisch gewünschte Position gewährleisten. Dabei ist eine Feineinstellung des Ausmaßes der Vorschubbewegung/ Kraft des Unterkiefers mit Hilfe des justierbaren Führungsbügels möglich.

Bei Geräten gemäß den Druckschriften US 5 848 891 A oder DE 100 42 049 B4 sind der Führungsbügel und/oder das Korrekturglied in der sagittalen und/oder um eine zur Sagittalebene im Wesentlichen orthogonalen Schwenkachse einstellbar. Die Behandlungseinheit muss nicht im sichtbaren Zahnbereich angebracht werden, so dass es im Verlauf einer Behandlung zu keinerlei ästhetischen Einbußen für den Patienten kommt. Die laterale Unterkieferbeweglichkeit kann durch eine transversal großzügige Justierung von Führungsbügel und Korrekturglied größer gestaltet werden als bei bekannten Ausführungsformen. Die Verwirklichung von Gleitflächen zwischen dem Führungsbügel und dem Korrekturglied mit jeweils vorgegebenem Neigungswinkel reduziert die Eingewöhnungszeit des Patienten auf ein Minimum und macht den therapeutischen Effekt von der Mitarbeit des Patienten unabhängig. Ferner ist eine physiologische Mundöffnungs- und Schließbewegung gewährleistet, so dass im Ganzen eine Verkürzung der Behandlungsdauer erreicht wird.

Diese Geräte sind insofern unbefriedigend, als ihr therapeutischer Effekt von einer Reihe von Rahmenbedingungen abhängig ist. Hierzu zählen beispielsweise die Kooperation und Compliance des Patienten. Erwartet wird das Eintreffen von wahrscheinlich wirkenden Erfolgen im Bereich des dentalen und skelettalen Gesichtsschädels. Treten diese Effekte gemäß Gauss'scher Verteilungskurve im Einzelfall nicht ein, so ist mit (Teil-) Misserfolg oder (Teil-) Rezidiv zu rechnen. Oft ist jedoch das muskuläre Trainingsprogramm aktiver Geräte so gut, das zwar muskulär eine therapeutische Zielposition erreicht werden kann, auch wenn dies skelettal und dental nicht vom Körper des Patienten umgesetzt wird. Hier wäre eine lange, ggf. lebenslange Kontrolle der muskulären Halteposition erforderlich.

Außerdem kennt man zur Therapie einer skeletttalen Rückbisslage des Unterkiefers festsitzende bimaxilläre Behandlungsmittel zur Bisslageeinstellung und/oder dentalen Kompensation von Dysgnathien, welche von der Mitarbeit des Patienten im Wesentlichen unabhängig sind. Dabei kann es sich um festsitzende federnde oder starre Geräte handeln, welche die Unterkieferbeweglichkeit einschränken und deren Anbringung im sichtbaren Zahnbereich erforderlich ist. Bei derartigen Geräten stört eine oft hohe Bruchanfälligkeit, erschwerte intraorale Aktivierung, eingeschränkte Mundöffnung sowie erschwerte Justierbarkeit und Patienteneingewöhnung.

Darüber hinaus sind auch sogenannte "Schnarchschienen" bekannt (US 2005/0028826 A1), die jedoch nur nachts benutzt werden und für einen Fachmann therapeutisch und technisch nichts mit der vorliegenden Erfindung und deren Funktion gemeinsam haben.

### Aufgabe und Vorteile der Erfindung

Der Erfindung liegt die Aufgabe zugrunde, eine kieferorthopädische Behandlungseinheit bzw. Retentionsvorrichtung zu schaffen, die sich durch eine patientenindividuelle und komfortable Bauweise auszeichnet. In vorteilhafter Weise kann insbesondere das individuelle Zusammenspiel von Dentition, Muskulatur und Gesichtsskelett, insbesondere der Kiefergelenkstrukturen, nach den Vorgaben der gnathologischen Lehre Berücksichtigung finden.

Zur Lösung dieser Aufgabe dienen die in Anspruch 1 aufgeführten (orthopedischen) Merkmale. Die in den Unteransprüchen aufgeführten Maßnahmen sind vorteilhafte Ausführungen und Weiterbildungen der Erfindung möglich. Dementsprechend zeichnet sich eine erfindungsgemäße Behandlungseinheit u.a. dadurch aus, dass die erste Führungsvorrichtung einschließlich des ersten, die erste Führungsfläche umfassenden Führungselementes als erste, einteiliges Bauelement ausgebildet ist und/oder dass die zweite Führungsvorrichtung einschließlich des zweiten, die zweite Führungsfläche umfassenden Führungselementes als zweites, einteiliges Bauelement ausgebildet ist.

Mit dieser Maßnahme wird u.a. erreicht, dass eine dauerhafte und erfolgreiche Behandlung bzw. Verbesserung/Beseitigung der individuellen Pathologie erfolgen kann. Gerade durch die dauerhafte Verwendbarkeit der Erfindung, d.h. über einen besonders langen Zeitraum bzw. Monate oder länger, wird auch bei besonders schwerwiegenden Fällen eine Verbesserung bzw. Heilung erreicht. Die vorteilhafte Einteiligkeit der Komponente für den Oberkiefer und die vorteilhafte Einteiligkeit der Komponente für den Unterkiefer führt zu einer besonders komfortablen Bauweise. Hier können nachteilige Ecken, Kanten, Ritzen, Holräume etc. wie beim Stand der Technik vermieden werden, was sich in Bezug auf die Mundhygiene bzw. Pflegbarkeit und Reinigungsfähigkeit ausgesprochen positiv auswirkt. So kann mit der Erfindung wirkungsvoll nachteilige Ablagerungen vermieden werden.

Es hat sich auch in ersten Versuchen gezeigt, dass ein einteiliges Bauelement gemäß der Erfindung besonders stabil bzw. robust ist und somit besonders lange hält bzw. stabil bleibt. Dies bedeutet zusätzlich ein großer Komfortgewinn für den Patienten, da eine mehrfache Montage bzw. Demontage (beschädigter bzw. beeinträchtigter) Behandlungseinheiten entfallen kann.

Generell kann das einteilige Bauelement bzw. die einteilige, künstliche Zahnkomponente gemäß der Erfindung beispielsweise mittels spanabhebender Herstellungsverfahren, z.B. Fräs-/Schleif-/ Polier- bzw. CNC-Bearbeitung eines metallischen Rohlings, oder auch mittels chemischen bzw. galvanischen Abscheide-Verfahren oder sogar mittels einem sogenannten "3D-Druck-Verfahren" oder dergleichen hergestellt werden.

In einer besonderen Weiterbildung der Erfindung sind das erste, einteilige Bauelement und/oder das zweite, einteilige Bauelement als Gusselement ausgebildet. Vorzugsweise sind das erste, einteilige Bauelement und/oder das zweite, einteilige Bauelement als künstliche Zahnkrone und/oder Teilkrone ausgebildet. Hiermit können bereits bewährte Techniken bzw. Verfahren der modernen Zahntechnik verwendet werden, was eine kostengünstige und qualitativ besonders hochwertige Umsetzung der Erfindung ermöglicht.

Gemäß der Erfindung ist das erste, die erste Führungsfläche umfassende Führungselement als im Wesentlichen in Richtung der Kieferbewegung ausgerichteter, bogenförmiger Führungsarm ausgebildet. Dieser vorteilhafte Führungsarm ermöglicht eine patientenindividuelle Führung des ersten Führungselementes bzw. Bauelementes mittels der ersten Führungsfläche entlang bzw. wenigstens teilweise im Kontakt mit der zweiten Führungsfläche des zweiten Führungselementes.

Gemäß der Erfindung ist das zweite, die zweite Führungsfläche umfassende Führungselement als im Wesentlichen quer oder zumindest teilweise senkrecht zur Richtung der Kieferbewegung ausgerichteter, bogenförmiger Führungsausleger ausgebildet. Hiermit kann ein vorteilhaft auskragender Arm bzw. Vorsprung generiert werden, der in vorteilhafter Wirkverbindung mit der ersten Führungsfläche steht, insb. dem Führungsarm des gegenüberliegenden Bauelementes/Zahnes.

Gemäß der Erfindung kann vor allem beim Schließen des Mundes bzw. Kiefers ein Wechselspiel der beiden einteiligen Bauelemente generiert werden, womit die Kieferbewegung geführt und in vorteilhafter Weise behandelt bzw. geheilt/verbessert wird.

Vorzugsweise weist der Führungsausleger an einem dem Zahn gegenüberangeordneten Ende eine Verdickung auf. Hiermit wird erreicht, dass dieses Ende Innenflächen des Mundes bzw. Gewebe nicht unangenehm drückt oder gar verletzt. Die vorteilhafte Verdickung am Ende des Auslegers führt zu einer weiteren Verbesserung des Tragekomforts der erfindungsgemäßen Behandlungseinheit.

Vorteilhafterweise ist der Führungsausleger spitzwinklig und/oder bogenförmig in oder entgegen der Richtung des Mundrachens ausgebildet. Hiermit kann eine vorteilhafte Führung bzw. Korrektur der pathologischen Gelenkbahnneigung erreicht werden. Beispielsweise hackt der Führungsarm bzw. die erste Führungsfläche in vorteilhafter Weise in den spitzwinklig ausgerichteten Ausleger gemäß der Erfindung ein und führt bzw. zieht die Kiefer bei der Gelenkbewegung in die anatomisch richtige bzw. bessere Position/Stellung.

Vorzugsweise ist die zweite, das zweite Führungselement umfassende

### - weiter auf Seite 7 der ursprünglich eingereichten Beschreibungsseiten -

Führungsvorrichtung an einem Zahn des Unterkiefers angeordnet. Es hat sich gezeigt, dass es von Vorteil ist, den in Kieferbewegung ausgerichteten, bogenförmigen Führungsarm am Oberkiefer und den quer zur Kieferbewegung ausgerichteten Führungsausleger am Unterkiefer anzuordnen.

Eine besonders stabile und langlebige Behandlungseinheit kann zumindest derzeit dadurch verwirklicht werden, dass das erste, einteilige Bauelement und/oder das zweite, einteilige Bauelement der Behandlungseinheit aus einem Metall oder einer Metalllegierung gegossen wird. Es können durchaus in Zukunft auch weitere Verfahren bzw. Materialien von besonderem Vorteil sein.

Generell kann gemäß der Erfindung eine festsitzende bimaxilläre, paarig anzubringende kieferorthopädisch-gnathologische Apparatur zur orthopädischen Ventralverlagerung des Unterkiefers sowie dessen Langzeitstabilisierung verwirklichen.

In die Konstruktion fließen in vorteilhafter Weise patientenindividuell für den passiven Zustand bei geschlossenem Mund ein: Beispielsweise die therapeutische Kiefergelenkszentrik, die therapeutisch gewünschte Unterkieferlage im Hinblick auf die muskuläre Haltung und die dentale Relation in Angle Klasse I.

Für die aktive Bewegung ist das Abbilden der Mundöffnungsbewegung in der Apparatur von großem Vorteil. Die Mundöffnungsbewegung wird determiniert durch die Kiefergelenksanatomie, insbesondere die Gelenkbahnneigung, sowie der Kiefergelenkpathologie, wie Diskusverlagerungen oder kondyläre Deformationen infolge von Erkrankungen oder Fehlbelastungen, beispielsweise rheumatoider Formenkreis.

Ebenso sind die Kriterien einer abgestützten Okklusion und einer Front-Eckzahnführung erfüllt, die Eingewöhnungszeit des Patienten auf ein Minimum zu beschränken und ein Langzeitverbleib zu ermöglichen.

Gemäß der Erfindung greifen die vorteilhaften Führungselemente, insbesondere der dauerstabile Führungsbügel/-arm des Oberkiefers und der daueratabile Führungsausleger des Unterkiefers, im therapeutisch gewünschten Zusammenbiss im Schlüssel-Schloss-Prinzip exakt ineinander. Bei aktiver Vorverlagerung ist vorzugsweise die Dentition über zusätzliche kieferorthopädische Maßnahmen zu schützen, um ungewünschte Zahnbewegungen zu vermeiden, die durch abgeleitete muskuläre Kräfte entstehen könnten. Dies ist meist nicht erforderlich, wenn der Patient bereits durch eine vorherige muskuläre Umstellung des Unterkiefers bereits in die Zielposition trainiert ist und nur eine muskuläre Entwöhnung verhindert werden soll.

Die Herstellung der erfindungsgemäßen Behandlungseinheit bzw. der beiden einteiligen Teilkomponenten/Bauelemente kann in vorteilhafter Weise analog der Herstellung einer Zahnkrone oder dergleichen, vorzugsweise nach gnathologischen Prinzipien erfolgen.

Zunächst sollte die gewünschte Kieferlage mittels Zahnmodellen in einen programmierbaren Artikulator übertragen werden. Der Artikulator ist auf die patientenindividuellen Parameter/Vorgaben der Gelenkbewegung einzustellen. Insbesondere Gelenkbahnneigung sowie Exkursionsbewegungen des Unterkiefers sind in vorteilhafter Weise einzustellen. Sowohl Protrusion als auch Laterotrusion des Patienten sollten mindestens möglich sein und daher in vorteilhafter Weise eingestellt werden.

Die insgesamt vier erfindungsgemäßen Bauelemente bzw. Verschlüsselungsteile, in der Regel auf beiden Seiten des Kiefers und/oder im nichtsichtbaren Bereich angebracht an den Zähnen 16, 26, 36, 46, werden in vorteilhafter Weise zahntechnisch hergestellt. Ein adäquates Verfahren ist hierbei das Vorgehen in einer zahntechnischen Aufwachstechnik mit nachfolgendem Guss der Rohlinge bzw. Bauelemente gemäß der Erfindung. Beim Aufwachsen der Bauteile bzw. Bauelemente sind die am Artikulator eingestellten patientenindividuellen Parameter/Vorgaben in vorteilhafter Weise in die Werkstücke bzw. Bauelemente einzuarbeiten. Die natürliche Dentition bietet in der Regel genügend Freiflächen im Bereich der Okklusal- und Approximalflächen, sodass es dem Zahntechniker möglich ist vorteilhafte Haltebereiche/-flächen für die Bauteile bzw. Bauelemente am entsprechenden Zahn zu finden, ohne den natürlichen Zusammenbiss zu stören. Im Einzelfall kann ein minimales zahnärztliches Einschleifen am Zahn erforderlich werden bzw. von Vorteil sein und die Anbringung bzw. die Fixierung am Zahn verbessern.

Zur Verwendung in der Herstellung können sämtliche zahntechnische Materialien kommen, vor allem Materialien, die zum vorteilhaften Langzeitverbleib im Mund geeignet sind. Hierzu zählen insbesondere Metalllegierungen, wie sie für prothetische Arbeiten Verwendung finden.

Somit ist durch die Apparatur nicht nur die aktive kieferorthopädische Behandlung möglich, insbesondere auch in Verbindung mit anderen Apparaturen, sondern im Fall einer unzureichenden Reaktionslage ist die Langzeitsicherung von maximalen Teilerfolgen ohne Apparaturenwechsel möglich. Auch beginnende Rezidive können mit Hilfe der Erfindung frühzeitig gestoppt und Behandlungsergebnisse erhalten werden.

### Ausführungsbeispiel

Ein Ausführungsbeispiel ist in der Zeichnung dargestellt und wird anhand der Figuren nachfolgend näher erläutert.

Im Einzelnen zeigt:
- Figur 1: schematisch eine korrekte anatomische Lage der Kiefer mit einer Behandlungseinheit gemäß der
- Figur 2: schematisch eine Anwendung der Erfindung bei einer Pathologie "Unzureichende Umbau-Reaktion des Kondylenkopfes nach Kieferorthopädie dadurch erweiterter Gelenkspalt",
- Figur 3: schematisch eine Anwendung der Erfindung bei einer Pathologie "Orthotope Diskuslage mit Höhenverlust durch Diskusperforation",
- Figur 4: schematisch eine Anwendung der Erfindung bei einer Pathologie "Stabilisierter Diskus bei unzureichender Reaktion des Kondylenkopfes nach Kieferorthopädie",
- Figur 5: schematisch eine Anwendung der Erfindung bei einer Pathologie "Erhalt einer erreichten skelettalen, dentalen und myofunktinellen Zentrik bei Diskusverlagerung",
- Figur 6: schematisch eine Anwendung der Erfindung bei einer Pathologie "Gelenkseitig instabile Situation mit erweitertem Gelenkspalt, nicht erreichter Bisslage und Diskusverlagerungen mit (Teil-) Reposition",
- Figur 7: schematisch eine Anwendung der Erfindung bei einer Pathologie "Gelenkseitig instabile Situation durch Kondylenresorption mit therapeutisch erweitertem Gelenkspalt unter Einhaltung der dentalen Klasse I",
- Figur 8: schematisch eine vergrößerte, seitliche Darstellung der Erfindung aus den vorherigen Figuren mit geöffnetem und (gestrichelt)
- Figur 9: schematisch eine vergrößerte Draufsicht der Erfindung aus den vorherigen Figuren vor und nach Laterotrusion.

In den Figuren ist schematisch eine kieferorthopädische Behandlungs- und Retentionseinheit zur Veränderung und dem Erhalt der Bisslage in einer therapeutisch gewünschten definierten und patientenindividuellen Lagebeziehung zwischen Oberkiefer (1) und Unterkiefer (2), mit einer Führungseinrichtung (3) zum temporären oder Langzeitverbleib insbesondere durch die Herstellung nach gnathologischer Lehre, mit einem anschließend an einem Oberkieferzahn befestigbaren dauerstabilen Führungsteil (4) und einem dauerstabilen Führungsbügels (6) zur Befestigung an einem Unterkieferzahn, derart ineinandergreifend dass bei einer Öffnungs- und Schließbewegung die natürlichen Kieferbewegungen über die Gleitflächen der Apparatur geführt werden in patientenindividueller Rotation, Protrusion und Laterotrusion, dargestellt, wobei die Führungsflächen nach patientenindividuellen Bewegungen auf die dauerstabilen Bauteile im Zuge deren Herstellung gestaltet sind.

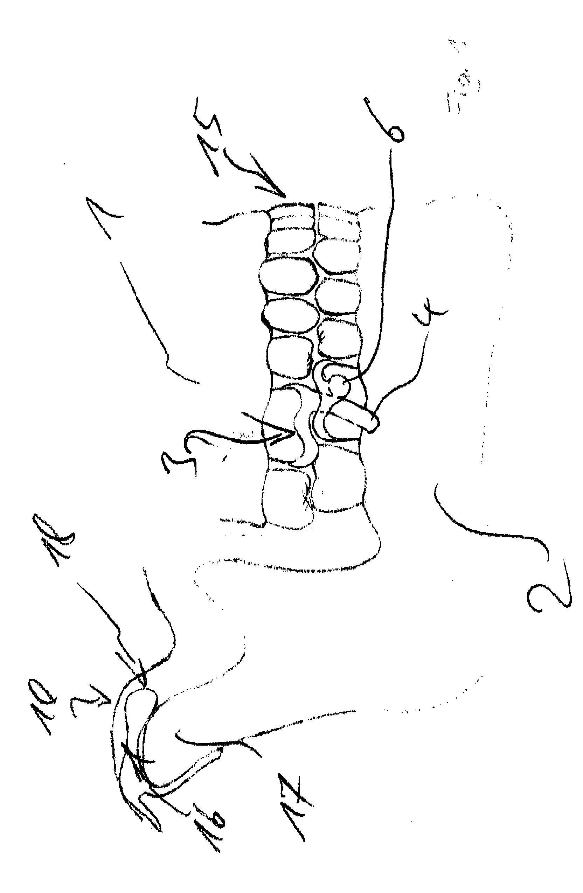

In Figur 1 ist zudem eine (patientenindividuell anatomisch vorgegebene) Gelenkbahn 10, ein (gleichförmiger) Gelenkspalt 16, ein zentrischer Kondylenkopf 17, eine orthotrope Diskuslage 18 sowie eine (patientenindividuell anatomisch vorgegebene) Front-Eckzahnführung 15 schematisch dargestellt.

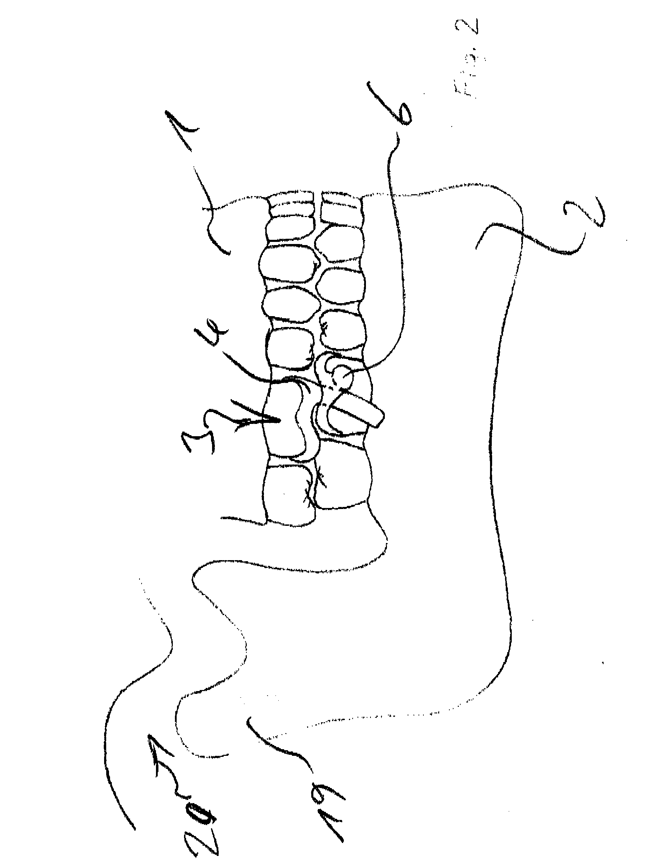

Figur 2 zeigt einen erweiterten Gelenkspalt 20 und eine anterior stehender Kondylenkopf 19 zum Erreichen der dental Klasse I bzw. eine unzureichende Reaktion des Kondylenkopfes 19 nach Kieferorthopädie, dadurch der erweiterte Gelenkspalt 20.

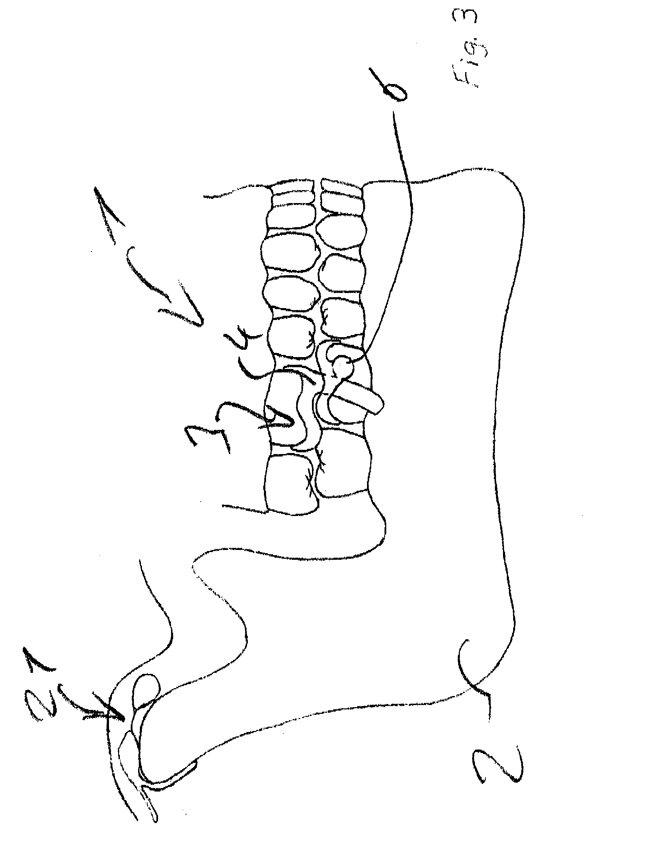

Figur 3 zeigt schematisch eine orthotope Diskuslage mit

Höhenverlust durch Diskusperforation bzw. ein perforierter Diskus 21.

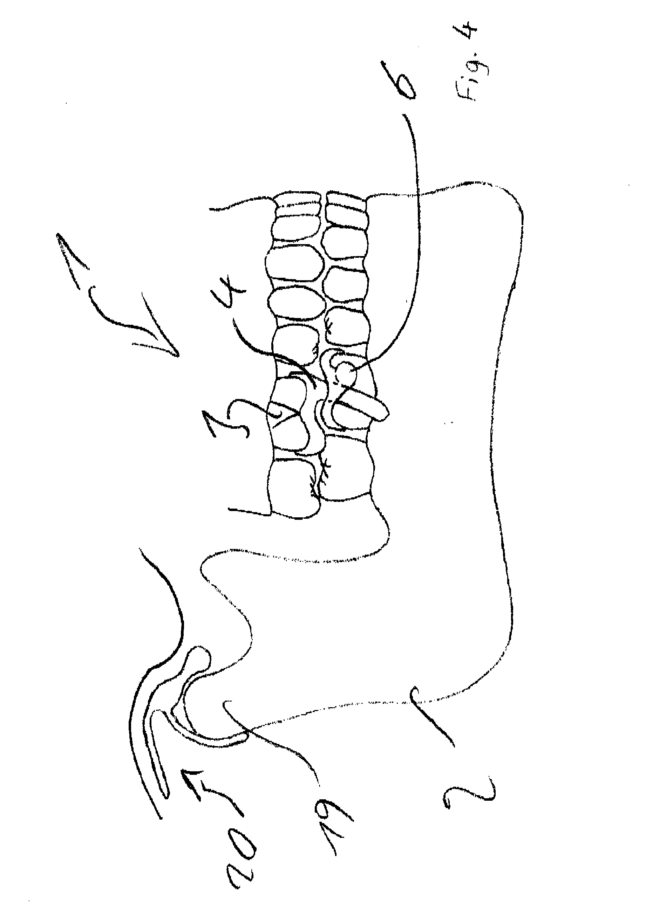

In Figur 4 ist schematisch ein stabilisierter Diskus 22 bei unzureichender Reaktion des Kondylenkopfes 19 nach Kieferorthopädie mit erweitertem Gelenkkopf 20 veranschaulicht.

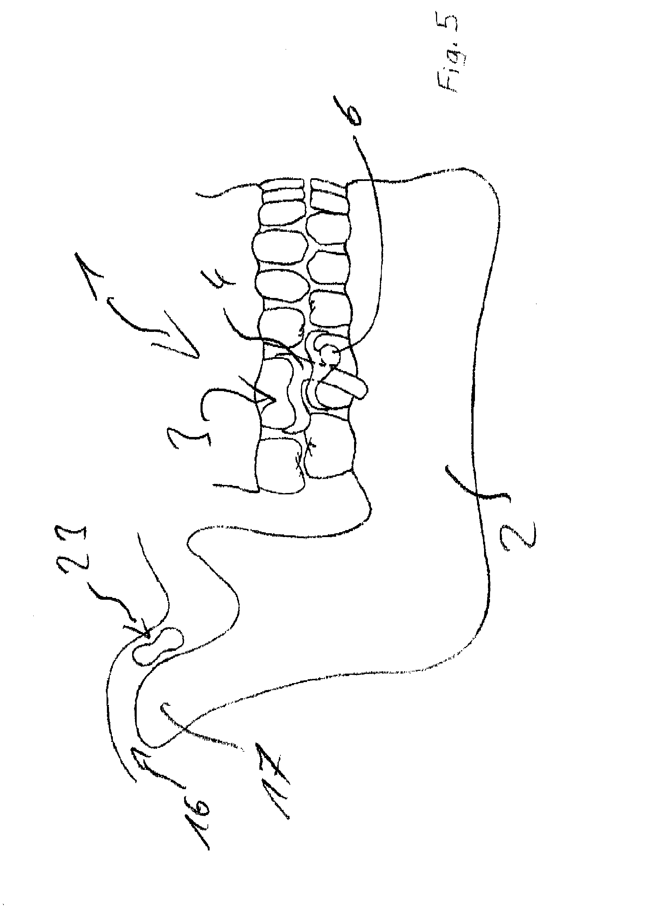

Figur 5 zeigt schematisch einen Erhalt einer erreichten skelettalen, dentalen und myofunktinellen Zentrik bei Diskusverlagerung 23, wobei ein zentrischer Kondylenkopf 17 und ein gleichförmiger Gelenkspalt 16 vorhanden ist.

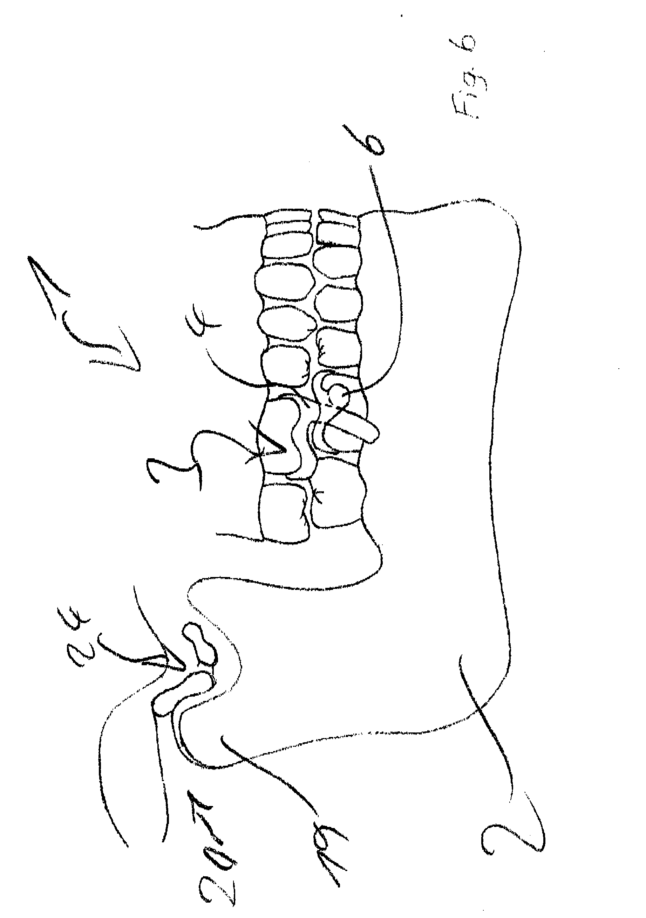

In Figur 6 ist schematisch eine gelenkseitig instabile Situation mit erweitertem Gelenkspalt 20, nicht erreichter Bisslage und Diskusverlagerungen mit (Teil-) Reposition dargestellt, wobei ein erweiterter Gelenkspalt 20, ein instabiler Diskus 24 sowie ein anterior stehender Kondylenkopf 19 zum Erreichen der dentalen Klasse I veranschaulicht ist.

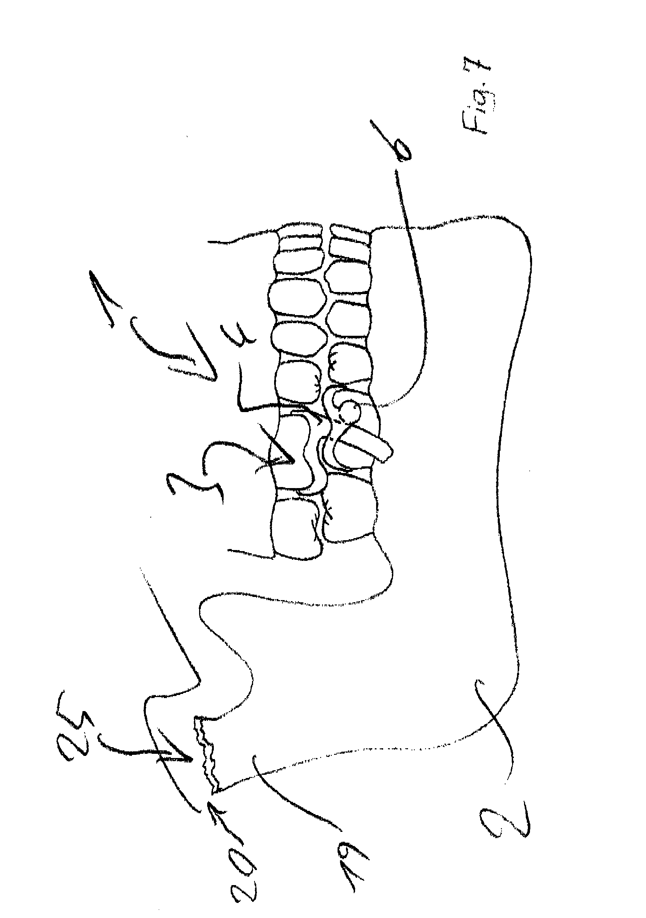

Figur 7 erläutert schematisch eine gelenkseitig instabile Situation durch Kondylenresorption mit therapeutisch erweitertem Gelenkspalt 20 unter Einhaltung der dentalen Klasse I, wobei ein anterior stehender Kondylenkopf 19 bzw. ein anterior eingestellter Kondylenkopf 25 mit Resorption und Beaking deutlich wird.

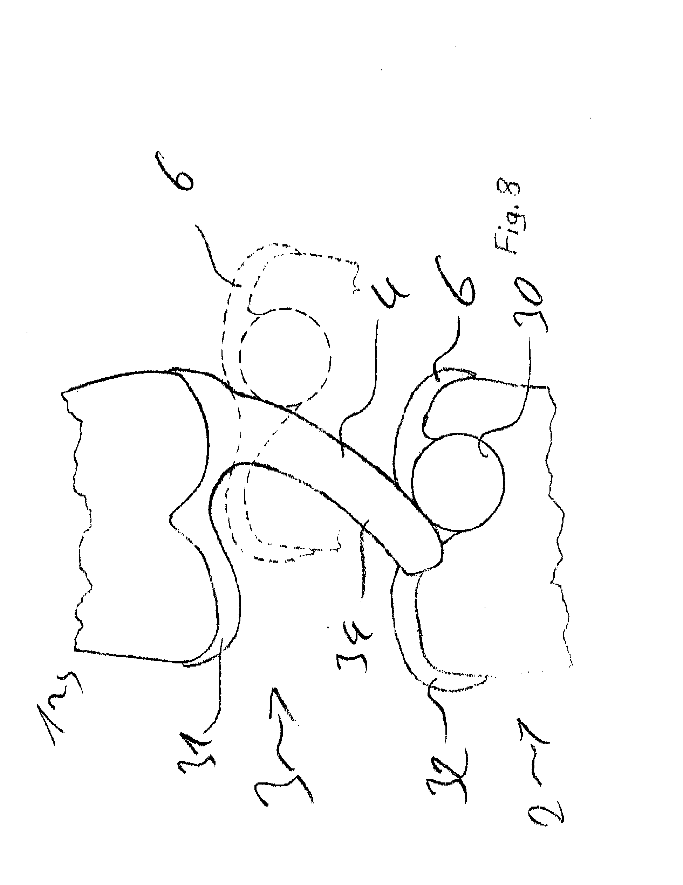

In Figur 8 ist schematisch eine vergrößerte, seitliche Darstellung der Behandlungseinheit 3 aus den vorherigen Figuren mit geöffnetem ur.d (gestrichelt) geschlossenem Kiefer 1, 2 abgebildet. Hierbei wird ersichtlich wie die Gelenkbahnbewegung eine vorteilhafte Führung der beiden einteiligen Bauelemente 4, 6 gewährleistet.

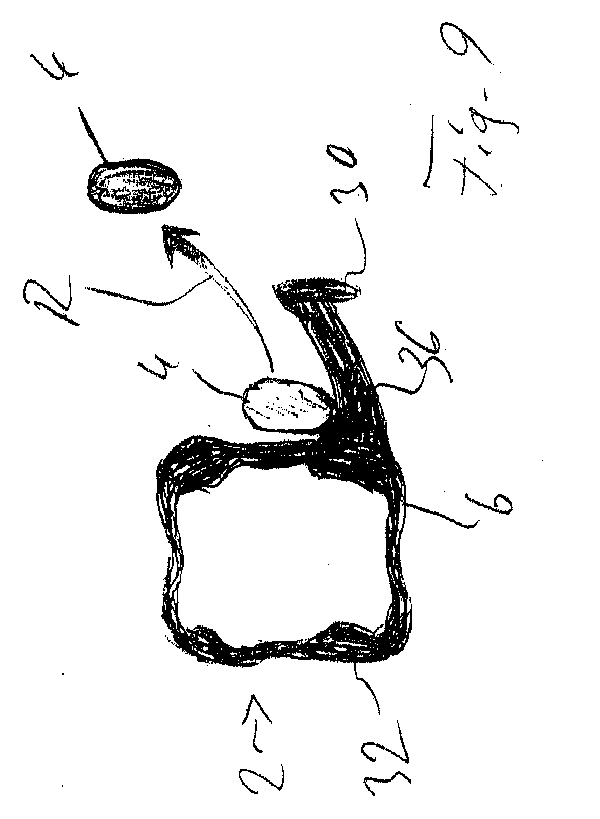

Figur 9 zeigt schematisch eine vergrößerte Draufsicht der Erfindung aus den vorherigen Figuren vor und nach Laterotrusion. Der Führungsbügel 6 bzw. Führungsausleger 6 steht quer zur Kieferbewegung und weist am Zahn abgewendeten Ende eine Verbreiterung 30 bzw. ein Schild 30 auf. Hiermit wird der Komfort für den Patienten verbessert.

Das Führungsteil 4 weist neben einem Zahnfixierbereich 31 zum Fixieren des Führungsteils 4 am Zahn zudem einen Führungsarm 34 auf, der bogenförmig und etwa in Richtung der Kieferbewegung bzw. der Schließbewegung gerichtet ist. Dieser Führungsarm 34 greift am anderen Teil 6 bzw. Ausleger 6, insbesondere einem Auslegerarm 36, ein und wird durch diesen bei zur geschlossenen Kieferstellung am Ausleger 6 und/oder Auslegerarm 36 geführt. Hierbei wird eine vorteilhafte Relativbewegung entlang der Richtung R bzw. entlang des schematisch dargestellten Pfeils R generiert. Das Teil 6 bzw. der Ausleger 6 weist einen Zahnfixierbereich 32 zum Fixieren des Führungsteils 6 am Zahn auf.

Gemäß der Erfindung ist das Führungsteil 4 und der Ausleger 6 bzw. deren Zahnfixierbereiche 31 oder 32 jeweils einteilig mit dem entsprechenden Arm 34 bzw. 36 ausgebildet, vorzugsweise als Metallguss hergestellt.

Es wird mit Hilfe der Figuren auch deutlich, dass die Behandlungseinheit 3 bzw. deren Oberflächen glatte rundliche Formen annehmen, insbesondere durch Hinzufügen eines Schutzschildes am äußeren Ende des seitlichen Auslegers, und Gewebeoberflächen weder durch Druck noch Penetration verletzbar sind. Aus den Figuren wird somit deutlich, dass die einteilige Bauweise der Komponenten 4, 6 dazu führt, dass eine Formgebung ohne Risse, Hohlräume, scharfe Kanten etc., sondern eine sehr runde, anschmiegsame Form gemäß der Erfindung realisierbar ist. Dies verbessert den Komfort sowie die Hygiene und zudem zu einem langen Verbleib der Erfindung im Mund bzw. am Zahn. Dies verbessert die Behandlung bzw. Heilung pathologischer Kiefer.

Durch die Verwendung vorteilhafter zahntechnischer Materialien ist ein bis zu lebenslanger Verbleib ermöglicht, insbesondere um Teilerfolge bei ungünstiger Reaktion langfristig zu sichern.

Vorteilhafter weise ist keine Veränderung der Zahnhartsubstanzen vorgesehen.

Aus den Figuren wird zudem ersichtlich, dass die zu reinigenden Übergänge zum natürlichen Zahn gut zugänglich sind.

## Patentansprüche

1. Kieferorthopädische Behandlungseinheit zur Veränderung bzw. Erhalt der Bisslage in einer therapeutisch gewünschten definierten und patientenindividuellen Lagebeziehung zwischen Oberkiefer (1) und Unterkiefer (2), wobei wenigstens eine Führungseinheit (3) zum temporären oder Langzeitverbleib am Oberkiefer (1) und Unterkiefer (2) vorgesehen ist, wobei die Führungseinheit (3) wenigstens eine erste, an einem ersten Zahn eines ersten Kiefers (1, 2) befestigbare Führungsvorrichtung (4) und eine zweite, an einem dem ersten Zahn zugeordneten, zweiten Zahn eines zweiten Kiefers (1, 2) befestigbare Führungsvorrichtung (6) umfasst, wobei die erste Führungsvorrichtung (4) ein erstes, eine erste Führungsfläche umfassendes Führungselement (34) zum Führen der zweiten Führungsvorrichtung (6) und wobei die zweite Führungsvorrichtung (6) ein zweites, eine zweite Führungsfläche umfassendes Führungselement (36) zum Führen der ersten Führungsvorrichtung (4) aufweist, so dass ein Ineinandergreifen des ersten und des zweiten Führungselementes (34, 36) bei einer Öffnungs- und Schließbewegung der Kiefer (1, 2) die natürlichen Kieferbewegungen in patientenindividueller Rotation, Protrusion und/oder Laterotrusion führen, wobei die erste Führungsvorrichtung (4) einschließlich des ersten, die erste Führungsfläche umfassenden Führungselementes (34) als erstes, einteiliges Bauelement (4) ausgebildet ist und/oder wobei die zweite Führungsvorrichtung (6) einschließlich des zweiten, die zweite Führungsfläche umfassenden Führungselementes (36) als zweites, einteiliges Bauelement (6) ausgebildet ist, wobei das erste, die erste Führungsfläche umfassende Führungselement (4) als im Wesentlichen in Richtung der Kieferbewegung ausgerichteter, bogenförmiger Führungsarm (34) ausgebildet ist und wobei das zweite, die zweite Führungsfläche umfassende Führungselement (36) als im Wesentlichen quer oder senkrecht zur Richtung der Kieferbewegung ausgerichteter, bogenförmiger Führungsausleger (36) ausgebildet ist.

2. Behandlungseinheit nach Anspruch 1, **dadurch gekennzeichnet, dass** das erste, einteilige Bauelement (4) als Gusselement (4) ausgebildet ist und/oder dass das zweite, einteilige Bauelement (6) als Gusselement (6) ausgebildet ist.

3. Behandlungseinheit nach einem der vorgenannten Ansprüche, **dadurch gekennzeichnet, dass** das erste, einteilige Bauelement (4) und/oder das zweite, einteilige Bauelement (6) als künstliche Zahnkrone (4, 6) und/oder Teilkrone (4, 6) ausgebildet sind.

4. Behandlungseinheit nach einem der vorgenannten Ansprüche, **dadurch gekennzeichnet, dass** der Führungsausleger (36) an einem dem Zahn gegenüberangeordneten Ende eine Verdickung (30) aufweist.

5. Behandlungseinheit nach einem der vorgenannten Ansprüche, **dadurch gekennzeichnet, dass** der Führungsausleger (36) spitzwinklig und/oder bogenförmig in Richtung des Mundrachens ausgebildet ist.

6. Behandlungseinheit nach einem der vorgenannten Ansprüche, **dadurch gekennzeichnet, dass** die zweite, das zweite Führungselement (36) umfassende Führungsvorrichtung (6) an einem Zahn des Unterkiefers (2) angeordnet ist.

7. Herstellungsverfahren zur Herstellung einer kieferorthopädischen Behandlungseinheit nach einem der vorgenannten Ansprüche, **dadurch gekennzeichnet, dass** die erste Führungsvorrichtung einschließlich des ersten, die erste Führungsfläche umfassenden Führungselementes als erste, einteiliges Bauelement ausgebildet wird und/oder dass die zweite Führungsvorrichtung einschließlich des zweiten, die zweite Führungsfläche umfassenden Führungselementes als zweites, einteiliges Bauelement ausgebildet wird.

8. Herstellungsverfahren nach dem vorgenannten Anspruch, **dadurch gekennzeichnet, dass** das erste, einteilige Bauelement und/oder das zweite, einteilige Bauelement der Behandlungseinheit aus einem Metall oder einer Metalllegierung gegossen wird.

9. Herstellungsverfahren nach einem der vorgenannten Ansprüche, **dadurch gekennzeichnet, dass** das erste, einteilige Bauelement und/oder das zweite, einteilige Bauelement der Behandlungseinheit und/oder eine Gussform nach patentienindividuellen Maßen und/oder Kieferbewegungen und/oder Gelenkbahnneigungen und/oder gnathologischen Parametern und/oder Vorgaben hergestellt wird.

## Claims

1. An orthodontic treatment unit for changing or preserving the biting position in therapeutically desired defined and patient-individualized positional relationship between upper jaw (1) and lower jaw (2), wherein at least one guide unit (3) is provided for the temporary or long-term disposition on the upper jaw (1) and lower jaw (2), wherein the guide unit (3) comprises at least a first guide device (4) which can be fastened to a first tooth of the first jaw (1, 2) and a second guide device (6) which can be fastened to a second tooth of a second jaw (1, 2) assigned to the first tooth, wherein the first guide device (4) has a first guide element (34) comprising a first guide surface for guiding the second guide device (6) and wherein the second guide device (6) has a second guide element (36) comprising a second guide surface for guiding the first guide device (4), so that the natural jaw movements guide a meshing of the first and the second guide element (34, 36) during an opening and closing movement of the jaw (1, 2) in patient-individualized rotation, protrusion and/or laterotrusion, wherein the first guide device (4) including the first guide element (34) comprising the first guide surface is designed as first one-piece component (4) and/or wherein the second guide device (6) including the second guide element (36) comprising the second guide surface is designed as second, one-piece component (6), wherein the first guide element (4) comprising the first guide surface is designed as arched guide arm (34) aligned substantially in the direction of the jaw movement and wherein the second guide element (36) comprising the second guide surface is designed as arched guide extension arm (36) aligned substantially transverse or perpendicular to the direction of the jaw movement.

2. A treatment unit according to Claim 1, **characterized in that** the first, one-piece component (4) is designed as cast element (4) and/or that the second, one-piece component (6) is designed as cast element (6).

3. A treatment unit according to any one of the aforementioned claims, **characterized in that** the first, one-piece component (4) and/or the second, one-piece component (6) are designed as artificial dental crowns (4, 6) and/ or partial crowns (4, 6).

4. A treatment unit according to any one of the aforementioned claims, **characterized in that** the guide extension arm (36) has a thickening (30) on an end arranged opposite the tooth.

5. A treatment unit according to one of the aforementioned claims, **characterized in that** the guide extension arm (36) is designed in an acute-angled and/or arched manner in the direction of the oropharynx.

6. A treatment unit according to any one of the aforementioned claims, **characterized in that** the second, guide device (6) comprising the second guide element (36) is arranged on a tooth of the lower jaw (2).

7. A production method for producing an orthodontic treatment unit according to any one of the aforementioned claims, **characterized in that** the first guide device including the first guide element comprising the first guide surface is designed as a first, one-piece component and/or that the second guide device including the second guide element comprising the second guide surface is designed as a second, one-piece component.

8. A production method according to the aforementioned claim, **characterized in that** the first, one-piece component and/or the second, one-piece component of the treatment unit is cast from a metal or a metal alloy.

9. A production method according to any one of the aforementioned claims, **characterized in that** the first, one-piece component and/or the second, one-piece component of the treatment unit and/or a casting mold is produced according to patient-individualized dimensions and/or jaw movements and/or condylar inclinations and/or gnathological parameters and/or specifications.

## Revendications

1. Unité de traitement orthodontique pour la modification ou le maintien de l'occlusion selon un rapport thérapeutiquement souhaité, défini et pour un patient individuel, entre la position de la mâchoire supérieure (1) et de la mâchoire inférieure (2), dans laquelle au moins une unité de guidage (3) est prévue pour un maintien, provisoire ou de longue durée, contre la mâchoire supérieure (1) et la mâchoire inférieure (2), l'unité de guidage (3) comprenant au moins un premier dispositif de guidage (2), pouvant être fixé à une première dent d'une première mâchoire (1, 2) et un deuxième dispositif de guidage (6), pouvant être fixé à une deuxième dent, affectée à la première dent, d'une deuxième mâchoire (1, 2), le premier dispositif de guidage (4) comprenant un premier élément de guidage (34), comprenant une première surface de guidage, pour guider le deuxième dispositif de guidage (6), et le deuxième dispositif de guidage (6) comprenant un deuxième élément de guidage (36), comprenant une deuxième surface de guidage, pour guider le premier dispositif de guidage (4), de telle sorte que, lors d'un mouvement d'ouverture et de fermeture des mâchoires (1, 2), les mouvements naturels de la mâchoire, en rotation, protrusion et/ou latérotrusion d'un patient individuel, conduisent à un engrènement du premier et du deuxième éléments de guidage (34, 36), le premier dispositif de guidage (4), y compris le premier élément de guidage (34), comprenant la première surface de guidage, étant conçu comme un composant (4) en une pièce, et/ou le deuxième dispositif de guidage (6), y compris le deuxième élément de guidage (36), comprenant la deuxième surface de guidage, étant conçu comme un deuxième composant (6) en une pièce, le premier élément de guidage (4), comprenant la première surface de guidage, étant conçu comme un bras de guidage (34) en forme d'arc, pour l'essentiel orienté dans la direction du mouvement des mâchoires, le deuxième élément de guidage (36), comprenant la deuxième surface de guidage, étant conçu sous forme d'une flèche de guidage (36) en forme d'arc, pour l'essentiel orientée perpendiculairement ou transversalement par rapport à la direction du mouvement des mâchoires.

2. Unité de traitement selon la revendication 1, **caractérisée en ce que** le premier composant (4) en une pièce est conçu comme un élément de coulée (4), et/ou que le deuxième composant (6) en une pièce est conçu comme un élément de coulée (6).

3. Unité de traitement selon l'une des revendications précédentes, **caractérisée en ce que** le premier composant (4) en une pièce et/ou le deuxième composant (6) en une pièce sont conçus comme des couronnes dentaires artificielles (4, 6) et/ou des couronnes partielles artificielles (4, 6).

4. Unité de traitement selon l'une des revendications précédentes, **caractérisée en ce que** la flèche de guidage (36) comprend un épaississement (30) à une extrémité opposée à la dent.

5. Unité de traitement selon l'une des revendications précédentes, **caractérisée en ce que** la flèche de guidage (36) est conçue avec un angle aigu et/ou en forme d'arc dans la direction de l'oropharynx.

6. Unité de traitement selon l'une des revendications précédentes, **caractérisée en ce que** le deuxième dispositif de guidage (6) comprenant le deuxième élément de guidage (36) est disposé contre une dent de la mâchoire inférieure (2).

7. Procédé de fabrication, pour fabriquer une unité de traitement orthodontique selon l'une des revendications précédentes, **caractérisé en ce qu'**on réalise le premier dispositif de guidage, y compris le premier élément de guidage, comprenant la première surface de guidage, comme un premier composant en une pièce, et/ou qu'on réalise le deuxième dispositif de guidage, y compris le deuxième élément de guidage, comprenant la deuxième surface de guidage, comme un deuxième composant en une pièce.

8. Procédé de fabrication selon la revendication précédente, **caractérisé en ce que** le premier composant en une pièce et/ou le deuxième composant en une pièce de l'unité de traitement sont coulés à partir d'un métal ou d'un alliage métallique.

9. Procédé de fabrication selon l'une des revendications précédentes, **caractérisé en ce que** le premier composant en une pièce et/ou le deuxième composant en une pièce de l'unité de traitement et/ou un moule de coulée, sont fabriqués selon les dimensions, et/ou les mouvements de la mâchoire et/ou des inclinaisons du guidage condylien et/ou des paramètres et/ou spécifications nathologiques d'un patient individuel.
